# EUROPÄISCHE PATENTSCHRIFT

(11) **EP 4 134 051 B1**
(45) Veröffentlichungstag und Bekanntmachung des Hinweises auf die Patenterteilung: **01.11.2023**
(21) Anmeldenummer: 22173571.5
(22) Anmeldetag: 16.05.2022
(51) Int. Cl.: A61F 7/00, A61J 3/00, A61M 13/00

(54) **DAMPFANWENDUNG UND VORRICHTUNGEN ZUR DAMPFANWENDUNG**
VAPOUR APPLICATION AND VAPOUR APPLICATION DEVICES
APPLICATION VAPEUR ET DISPOSITIFS D'APPLICATION VAPEUR

(30) Priorität: 19.05.2021 AT 503942021
(43) Veröffentlichungstag der Anmeldung: 15.02.2023
(73) Patentinhaber: Haslauer, Paul, 5020 Salzburg (AT)
(72) Erfinder: Haslauer, Paul, 5020 Salzburg (AT)
(74) Vertreter: Wildhack & Jellinek

(56) Entgegenhaltungen:
- EP-A1- 2 626 054
- EP-A2- 0 779 067
- DE-U1-202004 003 366
- DE-U1-202006 011 823
- KR-A- 20100 009 970

## Beschreibung

Die Erfindung betrifft eine Personenliege, insbesondere zur Verwendung bei einer Waldtherapie bzw. zur Verwendung bei der Prävention und Behandlung von Stress, Unruhezuständen, psychosomatischen Beschwerden, Kopfschmerzen und/oder Atemwegserkrankungen.

Das Bedampfen, Befeuchten oder Beduften von Räumen und Körpern wird häufig angewendet um das Wohlbefinden zu steigern. In einigen Fällen werden dabei Extrakte aus Pflanzen, z.B. ätherische Öle, zugesetzt, wie in den Patentschriften KR201009970, EP2626054 oder EP779067. Der Geruch der Extrakte hat dabei eine zusätzliche Wirkung auf Personen, die sich in dem bedampften, befeuchteten oder bedufteten Raum befinden. Allerdings gehen durch die Verarbeitung der Pflanzen zu Extrakten und vor allem auch durch die Lagerung der Extrakte viele wichtige Inhaltsstoffe verloren, so dass die natürliche Wirkung der Pflanzen nur sehr eingeschränkt genutzt werden kann. Dies gilt vor allem für Pflanzen, deren Inhaltsstoffe beispielsweise aufgrund unterschiedlicher chemischer Zusammensetzung, schwierig gemeinsam zu extrahieren und zu lagern sind, beispielsweise für Rindendrogen.

Aufgabe der Erfindung ist es daher die natürliche Wirkung von Pflanzen, insbesondere von Holzpflanzen, noch besser nutzen zu können. Insbesondere soll es ermöglicht werden, die positiven Effekte des Waldbadens ohne physischen Besuch eines Waldes zu erreichen.

Es wird daher vorgeschlagen, eine Bedampfung, Befeuchtung oder Beduftung von Räumen oder Körpern mit einem frisch zubereiteten Extrakt durchzuführen, wobei eine Rindendroge, vorzugsweise Rinde von Syzygium cumini, bereitgestellt wird, und wobei Wasserdampf durch oder über die Droge geleitet wird.

Die Natur hält viele Inhaltsstoffe bereit, die der Mensch zu den unterschiedlichsten Zwecken für sich nutzbar gemacht hat. Dabei werden aufgrund der langen Haltbarkeit häufig Drogen, also natürliche, insbesondere getrocknete, Substrate und Teile von Pflanzen, Harzen o.ä., verwendet. Erfindungsgemäß können die Inhaltsstoffe der Pflanzen besonders umfassend genutzt werden, da die gesamte Droge verwendet wird. Besonders bei Drogen, deren wirksame Inhaltsstoffe noch nicht vollständig erforscht sind, kann dadurch vermieden werden, dass wichtige Wirkstoffe bei der Extraktion und bei einer Qualitätskontrolle nach der Lagerung unbeachtet bleiben. Durch den Wasserdampf werden die Inhaltsstoffe freigesetzt. Die Extraktion der Wirkstoffe kann mit Nassdampf oder Heißdampf durchgeführt werden. Für Rindendrogen ist es besonders geeignet, wenn der Wasserdampf eine Temperatur von zumindest 100 °C aufweist. Die freigesetzten Inhaltsstoffe werden sofort zur Bedampfung verwendet. Der bei der Extraktion verwendete Dampf wird also unmittelbar für die Bedampfung des Körpers weiterverwendet. Es kann daher nicht zu Abbaureaktionen kommen, die bei einer Lagerung unweigerlich auftreten würden. Die Temperatur des für die Bedampfung verwendeten Wasserdampfs kann durch Zufuhr von kühlerer Umgebungsluft, beispielsweise abhängig von der verwendeten Droge und/oder der gewünschten Wirkung, reduziert und eingestellt werden. Für die Bedampfung eines Körpers wird Nassdampf verwendet. Wenn das Verfahren unter Verwendung von Rindendrogen durchgeführt wird, kann ein natürlicher Waldgeruch erzeugt werden, so dass der Eindruck des Waldbadens entsteht. Dabei wird das limbische System aktiviert. So können Ruhe und Gelassenheit gefördert werden. Auch andere positive Effekte, wie Prävention und Behandlung von Stress, Unruhezuständen, psychosomatischen Beschwerden, Kopfschmerzen, und/oder Atemwegserkrankungen können erzielt werden.

Besonders vorteilhaft ist ein Verfahren, wobei die Rinde von Syzygium cumini verwendet wird. Dieser Baum, der auch als Syzygium jambolana oder Jambulbaum bekannt ist, hat eine Vielzahl von unterschiedlichen Inhaltsstoffen.

Besonders geeignet ist die Verwendung von fermentierten und getrockneten Drogen, da dadurch die Wirksamkeit der Inhaltsstoffe und die Haltbarkeit der Droge verbessert werden kann. Dies kann beispielsweise erreicht werden, indem luftdurchlässige Säcke mit den geernteten losen Rinden auf Distanz-Roste mit leichter Luft-Durchdringung gelegt werden und eine langsame Trocknung der Rindenteilchen erfolgt. Die Schichtdicke kann beispielsweise ca. 20 cm betragen. Bei der Fermentierung werden die Inhaltsstoffe in der Droge gebunden.

Gerade für Syzygium cumini ist eine Fermentation der Rinde besonders geeignet, um die Haltbarkeit zu erhöhen.

Grundsätzlich ist es wichtig, dass es zu keiner Schimmelbildung in der Droge kommt, und dass keine Fremdkörper in der Droge enthalten sind. Es sollte daher bereits bei der Ernte auf hohe Qualität geachtet werden. Weiters sollte die Lagerung kühl und trocken erfolgen.

Zur besseren Freisetzung der Inhaltsstoffe kann vorgesehen sein, dass die Droge vor der weiteren Nutzung bzw. Bedampfung befeuchtet wird. Wenn die Droge zur Befeuchtung, insbesondere über mehrere Stunden, in Wasser eingelegt wird, so kommt es zunächst zu einer oberflächlichen Befeuchtung und die Freisetzung erfolgt langsamer und kontinuierlich. Beispielsweise kann die Droge zur Befeuchtung über 2 Stunden in warmem Wasser gelagert werden. Anschließend wird die Droge auf ein Sieb zum Abtropfen abgelegt.

Zum anderen können Inhaltsstoffe besonders gut freigesetzt werden durch eine Befeuchtung unter erhöhtem Druck mit Wasser oder Wasserdampf. Dabei kommt es zu einer tiefergehenden Befeuchtung und daher zu einer intensiveren Freisetzung der Wirkstoffe. Die Freisetzung der Wirkstoffe erfolgt zudem rascher.

Das zur Befeuchtung verwendete Wasser kann auch zur Dampferzeugung genutzt werden. Alternativ kann das zur Befeuchtung verwendete Wasser als Badezusatz verwendet werden.

Aufgabe ist es eine Vorrichtung bereitzustellen, die es ermöglicht, die natürliche Wirkung von Pflanzen, insbesondere Holzpflanzen, noch besser nutzen zu können und insbesondere eine Bedampfung möglichst zielgerichtet und einfach durchführen zu können.

Diese Aufgabe wird gelöst durch eine Personenliege gemäß Anspruch 1.

Die Personenliege umfasst eine Luftbefeuchtungsvorrichtung umfassend eine Wasserversorgungsvorrichtung, ein Heizelement zum Verdampfen des Wassers zur Erzeugung von Dampf, und einen Dampfauslass zur Abgabe des erzeugten Dampfs, wobei im Dampfauslass oder in Dampfströmungsrichtung direkt auf den Dampfauslass folgend ein Reservoir zur Aufnahme einer Droge vorgesehen ist. Es kann dabei vorgesehen sein, dass in dem Reservoir eine Droge, insbesondere eine Rindendroge, vorzugsweise Rinde von Syzygium cumini, angeordnet ist.

Diese Personenliege kann beispielsweise für ein zuvor beschriebenes Verfahren eingesetzt werden.

Im Rahmen der vorliegenden Anmeldung wird ein Reservoir dann als direkt auf den Dampfauslass folgend angesehen, wenn der von der Luftbefeuchtungsvorrichtung erzeugte Wasserdampf zumindest teilweise, insbesondere im Wesentlichen vollständig, durch das Reservoir geleitet wird.

Das Reservoir ermöglicht den Dampfeintritt zur Zuleitung des Dampfes auf die Droge und den Dampfaustritt zur Abgabe des mit den Inhaltsstoffen der Droge angereicherten Dampfs.

Wenn der Wasserdampf nur teilweise durch das Reservoir geleitet wird, so gelangt ein Teil des Dampfes in das Reservoir und wird mit den Inhaltsstoffen der Droge angereichert, während ein anderer Teil des Dampfes nicht durch das Reservoir geleitet wird. Dieser Teil, der nicht durch das Reservoir geleitet wird, hat eine höhere Strömungsgeschwindigkeit und zieht den mit Inhaltsstoffen angereicherten Teil des Dampfes mit. Dadurch kann es zu einer rascheren Verteilung des mit Inhaltsstoffen angereicherten Dampfes kommen.

Wenn der gesamte Dampf durch das Reservoir geleitet wird, kommt es zu einer stärkeren Anreicherung mit den Inhaltsstoffen der Droge, allerdings auch zu einer langsameren Verteilung des wirkstoffhaltigen Dampfes, da die Strömungsgeschwindigkeit des Dampfes durch die Droge stark verlangsamt wird.

Um eine Überhitzung der Luftbefeuchtungsvorrichtung zu vermeiden, kann vorgesehen sein, dass die Vorrichtung einen Trockengeh-Schutz aufweist, der zu einer automatischen Abschaltung führt, sobald das vorhandene Wasser vollständig verdampft ist.

Eine besonders einfache und effiziente Leitung des Dampfes durch die Droge wird ermöglicht, wenn das Reservoir als Beutel, insbesondere aus Organza, ausgebildet ist, wobei vorzugsweise vorgesehen ist, dass der Beutel am Dampfauslass reversibel abnehmbar befestigt ist.

Ein Beutel kann beispielsweise eine Größe von 40 x 30 cm aufweisen und mit ca. 250 g Rindendroge, insbesondere Rinde von Syzygium cumini, befüllt sein. Der Beutel sollte so um den Dampfaustritt angeordnet sein, dass die Droge gleichmäßig vom Dampf durchströmt wird.

Beispielsweise kann bei einer Luftbefeuchtungsvorrichtung mit einem stabförmigen Dampfauslass der Beutel auf den Dampfauslass gesteckt werden. Der Dampf tritt dann aus dem Dampfauslass und durchdringt das Reservoir und die darin enthaltene Droge, z.B. die darin enthaltene Rindendroge.

In diesem Fall wird aus dem Wasser aus der Wasserversorgungsvorrichtung durch das Heizelement Dampf erzeugt. Der Dampf tritt am Dampfauslass aus und trifft auf die im Reservoir angeordnete Droge. Der Dampf erwärmt das Wasser, das in der befeuchteten Droge eingelagert ist. Dieses Wasser verdampft nun ebenfalls und führt zur Freisetzung der Inhaltsstoffe der Droge, insbesondere auch zur Freisetzung der Wirkstoffe der Droge. Der wirkstoffhaltige Dampf verteilt sich im Raum und kann durch die Haut und die Atemwege aufgenommen werden.

Es kann auch vorgesehen sein, dass das Reservoir einen gitter- oder netzförmigen Dampfeintritt aufweist. Der Dampfeintritt kann in diesem Fall beispielsweise als Rost ausgebildet sein. In diesem Fall kann beispielsweise ein zuvor beschriebener Beutel über dem Rost, der in geringer Distanz zum Dampfauslass angeordnet ist, befestigt werden. Alternativ kann die Droge direkt auf dem gitter- oder netzförmigen Dampfeintritt angeordnet werden.

Es kann auch vorgesehen sein, dass in Dampfströmungsrichtung folgend ein reversibel verschließbarer Dampfaustritt mit einem, vorzugsweise dampfdichten, Verschluss vorgesehen ist. Wenn der Dampfaustritt durch den dampfdichten Verschluss verschlossen ist, kann die Droge, insbesondere die Rindendroge, direkt im Reservoir befeuchtet werden. Der in der Luftbefeuchtungsvorrichtung erzeugte Dampf gelangt dabei in das Reservoir. Da der Dampf nicht entweichen kann steigt der Druck. Es kann daher eine Befeuchtung unter erhöhtem Druck mit Wasserdampf durchgeführt werden. Die während der Befeuchtung freigesetzten Inhaltsstoffe der Droge werden nach der Befeuchtung durch Öffnen des Verschlusses freigesetzt und können somit auf einfache Weise genutzt werden.

Eine solche Luftbefeuchtungsvorrichtung kann beispielsweise eingesetzt werden zur Bedampfung, insbesondere des Körpers einer Person, wobei eine erfindungsgemäße Personenliege bereitgestellt wird, wobei die Luftbefeuchtungsvorrichtung gestartet wird, und wobei ein Körper auf der Liege positioniert wird. Die dampfdurchlässige Liegefläche ist insbesondere auch durchlässig für wirkstoffhaltigen Dampf, insbesondere wirkstoffhaltigen Nassdampf.

Besonders vorteilhaft ist es, wenn in einem solchen Verfahren vor dem Starten der Luftbefeuchtungsvorrichtung im Dampfauslass oder in Dampfströmungsrichtung direkt auf den Dampfauslass folgend eine Droge, insbesondere eine Rindendroge, vorzugsweise Rinde von Syzygium cumini, angeordnet wird.

Eine erfindungsgemäße Personenliege umfasst weiters eine dampfdurchlässige Liegefläche, wobei unterhalb der Liegefläche ein Dampfraum vorgesehen ist, wobei zwischen dem Dampfraum und der Liegefläche eine reversibel einstellbare Lamellenstruktur zur Steuerung des Dampfdurchtritts vom Dampfraum zur Liegefläche angeordnet ist, wobei die Lamellenstruktur kippbare Lamellen aufweist.

Die Menge des Dampfes bzw. des wirkstoffhaltigen Dampfes, die an den Körper gelangt, kann so reguliert werden. Dadurch wird vermieden, dass zu viel heißer Dampf an den Körper geleitet wird und es zu Verbrennungen kommt. Wenn die Lamellen geöffnet sind, so gelangt eine größere Menge an Dampf an den Körper, während durch Kippen der Lamellen die Menge reduziert werden kann.

Der Abstand zwischen Liegefläche und Dampfauslass sollte so gewählt werden, dass eine ausreichende Ablagerung des Dampfes auf dem Körper erfolgen kann, ohne jedoch zu Verbrennungen am Körper zu führen. Durch die Erwärmung des Körpers können beispielsweise Verspannungen im Becken -, Rücken -, oder Schulterbereich gelöst werden.

Besonders vorteilhaft ist es, wenn eine Luftbefeuchtungsvorrichtung, wie sie zuvor beschrieben wurde, vorgesehen ist. In diesem Fall kann eine besonders positive Wirkung durch die Erwärmung, die Inhaltsstoffe der Droge und den Geruch der Droge erzielt werden.

Für die Anordnung in einer Personenliege ist es besonders vorteilhaft, wenn die Luftbefeuchtungsvorrichtung so ausgebildet ist, dass der erzeugte Dampf teilweise durch die Droge geleitet wird. Der Teil des Dampfes, der durch die Droge geleitet wird, wird mit den Inhaltsstoffen der Droge angereichert, während der andere Teil des Dampfes nicht durch die Droge geleitet wird und aufgrund der höheren Strömungsgeschwindigkeit den mit Inhaltsstoffen angereicherten Teil des Dampfes mitzieht. Dadurch kann ein gleichmäßigere Bedampfung in allen Zonen der Liegefläche der Personenliege erreicht werden.

Eine besonders gezielte Bedampfung kann erfolgen, wenn die Lamellenstruktur entlang der Liegefläche mehrere Zonen aufweist, wobei die Zonen unabhängig voneinander einstellbar sind, wobei insbesondere vorgesehen ist, dass die Lamellenstruktur zumindest eine Kopfzone am oberen Ende der Liegefläche aufweist.

Um eine noch genauere Bedampfung zu ermöglichen, kann vorgesehen sein, dass an der Lamellenstruktur, insbesondere in jeder Zone der Lamellenstruktur, zumindest ein Sensor zur Messung der Temperatur des vorbeiströmenden Wasserdampfs vorgesehen ist, und wobei die Personenliege eine automatische Steuerung zur Einstellung der Lamellenstruktur anhand der gemessenen Temperatur aufweist.

Die Einstellung der Lamellenstruktur kann beispielsweise anhand eines Programms gesteuert werden.

Beispielsweise kann ein Wechsel von Bedampfungszeiten und bedampfungsfreien Zeiten vorgesehen sein. Eine weitere Möglichkeit ist es, dass beispielsweise im Kopfbereich nach einer vorgegebenen Zeit, insbesondere nach 10 Minuten, die Lamellen in die geschlossene Stellung gekippt werden, um eine Überwärmung zu vermeiden. Für eine besonders schonende Nutzung kann vorgesehen sein, dass der Kopf von der Bedampfung ausgenommen ist.

Zusätzlich oder alternativ ist auch eine Steuerung anhand von Sensoren möglich, wobei insbesondere vorgesehen ist, dass zumindest in der Kopfzone ein Sensor vorgesehen ist, wobei vorzugsweise vorgesehen ist, dass in jeder Zone ein Sensor vorgesehen ist.

Da im Kopfbereich eine Erwärmung nur für kurze Zeit toleriert wird, kann auch vorgesehen sein, dass bei der Erreichung eines vorgegebenen Temperaturschwellenwertes die Lamellen im Kopfbereich in die geschlossene Stellung gekippt werden, um eine Überwärmung des Kopfes zu vermeiden, ohne dass die Behandlung anderer Körperstellen abgebrochen werden muss.

In der Beckenzone kann beispielsweise eine größere Menge an Dampf zur Liegefläche geleitet werden, als dies in der Kopfzone gewünscht ist.

Um die Temperatur des Dampfs, der durch die Liegefläche tritt, besonders einfach steuern zu können, kann vorgesehen sein, dass ein einstellbarer Lufteinlass zum Einlass von Umgebungsluft in den Dampfraum vorgesehen ist, wobei insbesondere vorgesehen ist, dass die Personenliege eine automatische Steuerung zur Einstellung des Lufteinlasses aufweist, wobei vorzugsweise vorgesehen ist, dass die Einstellung des Lufteinlasses anhand der im Dampfraum oder an der Lamellenstruktur gemessenen Temperatur erfolgt.

Dadurch kann die Temperatur des Dampfstromes der durch die Liegefläche tritt noch besser reguliert werden, da durch den Lufteinlass kühlere Umgebungsluft in den Dampfraum eindringen kann.

Allerdings wird dadurch die Konzentration der Wirkstoffe im Dampfraum reduziert. Um dies zu vermeiden, kann auch vorgesehen sein, dass im Dampfraum, insbesondere in der Unterschenkelzone des Dampfraums ein Ventilator vorgesehen ist. Dadurch kann der kühlere Dampf bzw. die kühlere Luft aus der Unterschenkelzone nach oben befördert werden und so zu einer Abkühlung des Dampfstromes in anderen Zonen beitragen.

Die Größe des Dampfraumes und damit die benötigte Menge an Dampf kann reduziert werden, wenn die Personenliege dampfundurchlässige Seitenteile zur Begrenzung des unter der Liegefläche angeordneten Dampfraums aufweist. Dadurch kann der Dampfraum effektiv von der Umgebungsluft getrennt sein und die Konzentration und Wirkung des Dampfes auf den Körper kann verstärkt werden.

Zusätzlich oder stattdessen kann vorgesehen sein, dass die Personenliege eine der Liegefläche gegenüberliegende dampfundurchlässige Unterseite zur Begrenzung des Dampfraums aufweist. Besonders vorteilhaft ist es, wenn die dampfundurchlässige Unterseite beheizbar ist. Dadurch kann die Bildung von Schimmel im Dampfraum vermieden werden und die Personenliege kann auch als trockene Wärmeliege genutzt werden.

Um die Bedampfung noch effektiver zu machen, kann vorgesehen sein, dass oberhalb der Liegefläche ein mit dem Dampfraum in Verbindung stehender weiterer Dampfraum ausgebildet ist, wobei eine Abdeckung zur zumindest teilweisen Abgrenzung des weiteren Dampfraums von der Umgebungsluft vorgesehen ist.

Durch die Abdeckung kann die Wirkung der Bedampfung verstärkt werden, da ein ungewolltes Entweichen des Dampfes vermieden werden kann. Dadurch wird einerseits das Schwitzen verstärkt und andererseits auch die Aufnahme der Inhaltsstoffe in den Körper. Es kommt zu einer stärkeren Erwärmung des Körpers und zu einer höheren Konzentration der Inhaltsstoffe der Droge am Körper.

Um eine bessere Verteilung des wirkstoffhaltigen Dampfes im weiteren Dampfraum zu ermöglichen kann vorgesehen sein, dass im weiteren Dampfraum zumindest ein Ventilator angeordnet ist.

Die Abdeckung kann entweder über dem gesamten Körper vorgesehen sein, oder nur in bestimmten Körperbereichen, beispielsweise im Becken- und Oberschenkelbereich. Eine Abdeckung des Kopfbereiches kann ebenfalls erfolgen, sodass die Konzentration der eingeatmeten Inhaltsstoffe erhöht ist. Eine Abdeckung im Kopfbereich sollte jedoch nach ca. 10 Minuten entfernt werden. Es kann auch vorgesehen sein, dass die Abdeckung den Kopfbereich ausspart bzw., dass die Abdeckung den Körper vom Halsbereich abwärts abdeckt. Dadurch kann eine besonders schonende Nutzung erfolgen, die auch für Personen, die zu Kopfschmerzen oder Migräne neigen, gut verträglich ist.

Als Abdeckung kann ein Laken oder eine Abdeckfolie dienen, wobei jedoch zwischen Liegefläche und Abdeckung ein ausreichender Abstand einzuhalten ist um eine Berührung eines auf der Liegefläche liegenden Nutzers zu vermeiden. Dazu können Distanzhalter vorgesehen sein.

Alternativ kann eine stabile Abdeckung vorgesehen sein, um den Abstand zwischen Liegefläche und Abdeckung sicherzustellen. Die Abdeckung kann dann, beispielsweise entlang einer Schiene oder mit Hilfe von Distanzhaltern, im gewünschten Abstand zur Liegefläche angeordnet werden kann.

Um eine Bedampfung jederzeit abbrechen zu können, kann vorgesehen sein, dass die Abdeckung von innen entfernbar bzw. zu öffnen ist.

Die Abdeckung kann Zonen aufweisen die eine Öffnung der Abdeckung unabhängig von den anderen Zonen ermöglichen, wobei die Zonen der Abdeckung den Zonen der Lamellenstruktur entsprechen können, sodass die Bedampfung der Körperbereiche noch besser reguliert werden kann. Beispielsweise ist der Becken- und Oberschenkelbereich wenig wärmeempfindlich, während Bauch -, Brust -, und Nackenbereich wärmeempfindlicher sind.

Dabei kann vorgesehen sein, dass die Abdeckung zumindest eine einstellbare Abdeckungsöffnung zum Luftaustausch mit der Umgebung aufweist, wobei insbesondere vorgesehen ist, dass die Abdeckungsöffnung eine automatische Steuerung zur Einstellung der Abdeckungsöffnung aufweist, wobei vorzugsweise vorgesehen ist, dass die Einstellung der Abdeckungsöffnung anhand der an der Abdeckung gemessenen Temperatur erfolgt. Um eine Verteilung der durch die Abdeckungsöffnung ein- bzw. ausströmenden Luft zu verbessern, kann im Bereich der Abdeckungsöffnung ein Ventilator vorgesehen sein.

Wenn eine Bedampfung bzw. Beduftung der Umgebung der Personenliege vermieden werden soll, so kann in der Abdeckungsöffnung ein Filter eingebaut werden. Die Personen liege ist in diesem Fall in allen Räumlichkeiten nutzbar und kann beispielsweise auch im Schlafzimmer genutzt werden. Weiters ist dadurch auch eine Nutzung mit unterschiedlichen Gerüchen bzw. unterschiedlichen Drogen in kurz aufeinanderfolgenden Zeiträumen möglich, sodass die Personen liege innerhalb kurzer Zeiträume von mehreren Personen individuell abgestimmt genutzt werden kann.

Für eine gemeinsame Nutzung durch zwei Personen kann auch eine Personenliege mit zwei, insbesondere getrennt einstellbaren, Liegebereichen vorgesehen sein.

Um eine besonders vielseitige Nutzung zu ermöglichen, kann vorgesehen sein, dass die Luftfeuchtigkeit und/oder die Lufttemperatur im weiteren Dampfraum einstellbar ist, wobei insbesondere vorgesehen sein, kann, dass die Luftfeuchtigkeit bei etwa 5 % bis 100 % und/ oder die Lufttemperatur bei 45 ° bis 100° C liegt. Die Personenliege kann dann als Dampfbadliege, trockene Wärmeliege oder Saunaliege verwendet werden. Dazu kann beispielsweise die Menge des Wasserdampfs reguliert werden. Auch eine Regulierung der Temperatur an der Liegefläche und/oder der dampfundurchlässigen Unterseite kann vorgesehen sein. Beispielsweise kann bei Erzeugung einer geringen Menge an Wasserdampf die Liege als Saunaliege genutzt werden. Dazu wird die Luftfeuchtigkeit im weiteren Dampfraum auf etwa 10 % bis 30 % eingestellt und die Lufttemperatur auf etwa 60° C bis zu 100 °C. Um eine besonders genaue Regulierung zu erreichen, kann vorgesehen sein, dass zumindest ein Luftfeuchtigkeitssensor im weiteren Dampfraum vorgesehen ist. Bei der Nutzung als Wärmeliege kann die Temperatur beispielsweise auf etwa 45 °C bis 60 °C eingestellt werden.

Um zu vermeiden, dass es zu Verbrennungen durch Heißdampf kommt, kann im Dampfraum eine Trennwand vorgesehen sein, die den direkten Strömungsweg vom Dampfaustritt zur Liegefläche unterbricht, sodass ein längerer Strömungsweg des Dampfes vorgegeben wird, der ein Abkühlen des Dampfes bewirkt.

Um eine besonders schonende und gleichzeitig sehr effektive Erwärmung des Körpers zu erreichen, kann vorgesehen sein, dass der Kopfbereich von einer Bedampfung oder Erwärmung ausgenommen werden kann, bzw. das im Kopfbereich die Temperatur und/oder die Luftfeuchtigkeit gesondert einstellbar ist. Auf diese Weise können Kopfschmerzen nach einer Behandlung vermieden werden. Auch empfindliche Patienten, beispielsweise Patienten mit Migräne, können die Personenliege somit nutzen.

Auch eine Nutzung als Ruheliege ist möglich. Es kann also mit vergleichsweise geringem Platzbedarf und geringen Betriebskosten eine Vielzahl an Anwendungsmöglichkeiten vereint werden.

Eine weitere Anwendungsmöglichkeit besteht darin, eine neue schonende Therapieoption bereitzustellen, insbesondere bei Stress, Unruhezuständen, psychosomatischen Beschwerden, Kopfschmerzen, Atemwegserkrankungen und/oder Diabetes mellitus.

Dazu kann ein frischer Wasserdampfextrakt einer Rindendroge, insbesondere den Extrakt der Rinde von Syzygium cumini, zur Verwendung als Arzneimittel bereitgestellt werden, wobei der Extrakt durch Wasserdampfextraktion gewonnen wird und unmittelbar nach der Gewinnung ein Raum in dem sich eine Person befindet und/oder zumindest ein Bereich des Körpers eines Patienten mit dem Extrakt bedampft werden. Die Herstellung bzw. Gewinnung des Extrakts kann wie eingangs beschrieben erfolgen. Dabei ist es besonders vorteilhaft, wenn die Wasserdampfextraktion unter Druck erfolgt. Für die Bedampfung wird vorzugsweise Nassdampf verwendet, wobei die Temperatur vorzugsweise durch Zufuhr von Umgebungsluft reduziert werden kann, um Verbrennungen zu vermeiden.

Besonders effektiv ist der frische Wasserdampfextrakt einer Rindendroge zur Verwendung bei einer Waldtherapie. Der frische Wasserdampfextrakt kann auch zur Verwendung bei der Prävention und Behandlung von Stress, Unruhezuständen, psychosomatischen Beschwerden, Kopfschmerzen, und/oder Atemwegserkrankungen bereitgestellt werden.

Eine spezielle Wirkung hat der frische Wasserdampfextrakt der Rinde von Syzgyium cumini zur Verwendung bei der Behandlung von Diabetes mellitus.

Es hat sich eine Senkung der morgendlichen Nüchtern-Blutzuckerwerte gezeigt, die eine Reduktion der Einnahme blutzuckersenkender Medikamente ermöglichte.

Um die Wirkung des frischen Wasserdampfextrakts einer Rindendroge effektiv, einfach und schonend zu nutzen kann der Extrakt zur Verwendung als Arzneimittel, insbesondere bei der Behandlung von Stress, Unruhezuständen, psychosomatischen Beschwerden, Kopfschmerzen, Atemwegserkrankungen und/oder Diabetes mellitus, bereitgestellt werden, wobei die Bedampfung zumindest einmal täglich erfolgt, insbesondere genau einmal täglich. Besonders einfach und effektiv ist es, wenn die Bedampfung zumindest einmal täglich erfolgt, insbesondere genau einmal täglich.

Die Anwendung kann für 10 bis 20 Minuten erfolgen. Es kann auch vorgesehen sein, dass die Dauer nach dem Bedarf bzw. dem Befinden des Patienten angepasst wird.

Besonders effektiv ist eine Anwendung, wenn während einer Bedampfung verschiedene Zonen des Körpers unterschiedlich lange bedampft werden. Es kann auch vorgesehen sein, dass während einer Bedampfung zumindest ein Körperbereich im Intervall mit bedampfungsfreien Zeiten bedampft wird.

Weiters kann zur Verstärkung und Verlängerung der Wirkung vorgesehen sein, dass nach dem Ende der Bedampfung die Auftragung einer topischen Zubereitung auf der bedampften Körperstelle erfolgt. Dadurch werden die auf der Haut abgelagerten Wirkstoffe zusammen mit der Zubereitung in die Haut aufgenommen.

Besonders schonend und effektiv ist der frischer Wasserdampfextrakt einer Rindendroge zur Verwendung, wobei die Bedampfung des Körpers auf einer zuvor beschrieben Personenliege erfolgt. i. Dabei ist es besonders vorteilhaft, wenn die Personenliege eine Abdeckung aufweist, da dadurch die Intensität der Bedampfung verstärkt werden kann.

Der frische Wasserdampfextrakt einer Rindendroge, insbesondere der Rinde von Syzgyium cumini, ist besonders geeignet zur Verwendung bei der Behandlung von Diabetes mellitus, wobei die Anwendungen präoperativ zur Blutzuckersenkung erfolgt. Es kann dadurch eine Verschiebung von Operationen aufgrund zu hoher Blutzuckerwerte vermieden werden. Dies kann helfen, dass medizinisch wichtige Operationen auch bei Patienten mit Diabetes mellitus zeitnah durchgeführt werden können.

Die Erfindung ist anhand der folgenden Figuren und des folgenden Beispiels ohne Einschränkung des erfinderischen Gedankens beispielhaft beschrieben.
Fig. 1 zeigt eine beispielhafte Luftbefeuchtungsvorrichtung.
Fig. 2 zeigt eine beispielhafte Personenliege.
Fig. 3 zeigt eine beispielhafte Lamellenstruktur.
Fig. 4 zeigt eine weitere beispielhafte Personenliege mit einer Abdeckung.

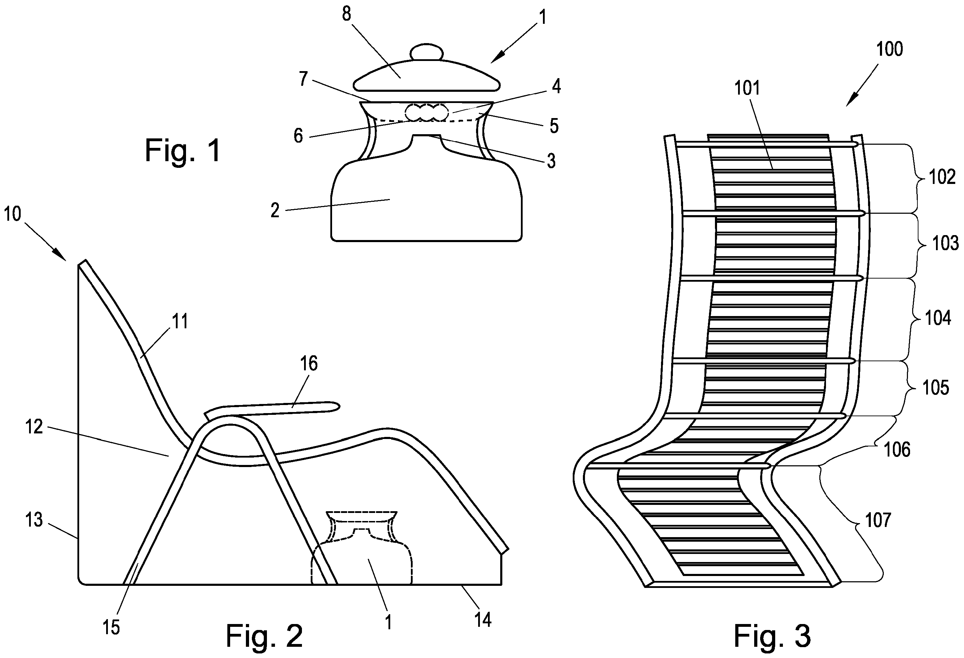

Fig. 1 zeigt eine beispielhafte Luftbefeuchtungsvorrichtung 1. Die Luftbefeuchtungsvorrichtung 1 weist eine Wasserversorgungsvorrichtung 2 auf und ein nicht dargestelltes Heizelement zur Erzeugung von Wasserdampf. Weiters ist ein Dampfauslass 3 zur Abgabe des Wasserdampfs vorgesehen. Direkt über dem Dampfauslass 3 ist als Reservoir 5 eine Schale vorgesehen, die einen Bodenrost als Dampfeintritt 6 und zur Auflage einer Droge 4 aufweist.

Das Reservoir 5 wird seitlich so gehalten, dass der aus dem Dampfauslass 3 austretende Dampf vollständig durch das Reservoir 5 geleitet wird. Es ist daher möglich, die Droge 4 im Reservoir 5 so anzuordnen, dass der gesamte erzeugte Dampf durch die Droge 4 geleitet wird oder nur ein Teil des erzeugten Dampfes.

In der dargestellten Ausführungsform ist die Droge 4 nur in einem Bereich des Reservoirs 5 angeordnet, insbesondere möglichst nahe des Dampfauslasses 3, im vorliegenden Beispiel also mittig. In diesem Fall wird ein Teil des erzeugten Dampfes durch die Droge 4 geleitet, während ein anderer Teil des Dampfes an der Droge 4 vorbei durch das Reservoir 5 strömt. Die Strömungsgeschwindigkeit des Dampfes, der durch die Droge 4 strömt, wird stark vermindert, während die Strömungsgeschwindigkeit des anderen Teils kaum beeinflusst wird. Der an der Droge 4 vorbeiströmende Teil des Dampfes zieht daher den durch die Droge 4 strömenden Teil des Dampfes mit, sodass es zu einer raschen Verteilung des mit Inhaltsstoffen angereicherten Dampfes kommt.

In einer nicht dargestellten Ausführungsform kann statt der Schale auch ein Beutel, beispielsweise mit einem Durchmesser von 30 cm, als Reservoir 5 verwendet werden. Besonders bevorzugt ist der Beutel aus Organza. Der Beutel kann ebenfalls seitlich so gehalten werden, dass der aus dem Dampfauslass 3 austretende Dampf entweder vollständig oder nur teilweise durch die Droge 4 geleitet wird.

In der dargestellten Ausführungsform weist das Reservoir 5 zusätzlich einen Dampfaustritt 7 auf, der durch einen dampfdichten Verschluss 8, abgedeckt werden kann. Dadurch in der Luftbefeuchtungsvorrichtung 1 eine Befeuchtung der Droge 4 mit Wasserdampf bei erhöhtem Druck erfolgen.

Als Droge 4 ist in der dargestellten Ausführungsform die getrocknete und fermentierte Rinde von Syzygium cumini im Reservoir 5 angeordnet.

Fig. 2 zeigt eine beispielhafte Personenliege 10 zur Bedampfung. Die Personen liege 10 weist eine Liegefläche 11 auf, die durch Beine 15 verkippbar gelagert ist. Weiters sind am oberen Ende der Beine 15 der Personenliege 10 Armlehnen 16 angeordnet. Unterhalb der Liegefläche 11 ist ein Dampfraum 12 ausgebildet. Der Dampfraum 12 wird seitlich von dampfundurchlässigen Seitenteilen 13 und unten von einer dampfundurchlässigen Unterseite 14 begrenzt. Im Dampfraum 12 ist eine Luftbefeuchtungsvorrichtung 1 angeordnet, die in der dargestellten Ausführungsform der Luftbefeuchtungsvorrichtung 1 aus Fig. 1 entspricht.

Fig. 3 zeigt eine beispielhafte Lamellenstruktur 100, die unter der Liegefläche 11 einer Personenliege 10 angeordnet werden kann. Die Lamellenstruktur 100 weist einstellbare Lamellen 101 auf, die durch Verkippen geschlossen oder geöffnet werden können, wobei eine stufenlose Einstellung der Öffnung ermöglicht ist. Die Lamellenstruktur 100 weist unterschiedliche Zonen 102, 103, 104, 105, 106, 107 auf, die unabhängig von einander eingestellt werden können. Am oberen Ende der Lamellenstruktur 100 ist eine Kopfzone 102 angeordnet, darunter folgt eine Schulter-/Nackenzone 103, eine Rückenzone 104, eine Beckenzone 105, eine Oberschenkelzone 106 und eine Unterschenkel -/Fußzone 107. Diese Zonen 102, 103, 104, 105, 106, 107 können so unterhalb der Liegefläche 11 angeordnet werden, dass die entsprechenden Körperbereiche gezielt bedampft werden können.

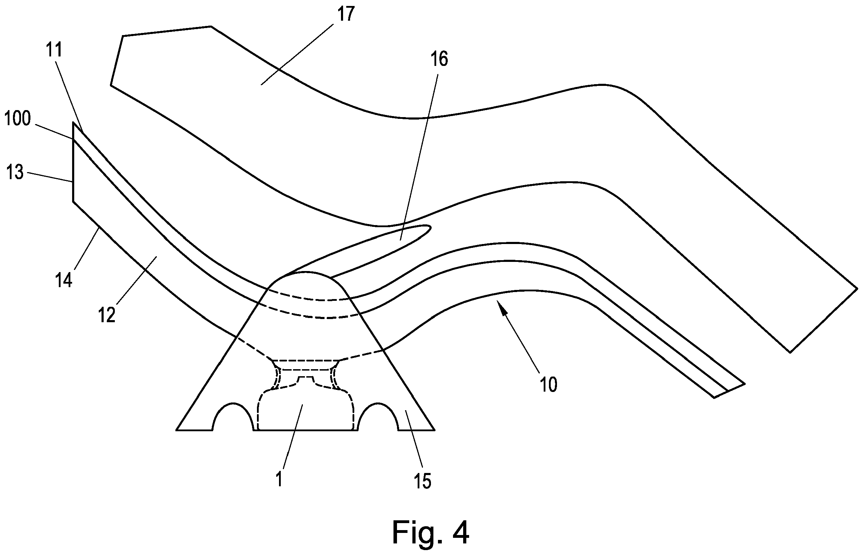

Fig. 4 zeigt ebenfalls eine beispielhafte Personenliege 10. Diese weist grundsätzlich den gleichen Aufbau auf, wie die zu Fig. 2 beschriebene Personenliege 10. Zusätzlich ist in diesem Fall bereits eine Lamellenstruktur 100, wie zu Fig. 3 beschrieben, unterhalb der Liegefläche 11 befestigt, sodass Dampf aus dem Dampfraum 12 durch die Lamellenstruktur 100 zur Liegefläche 11 strömt. Zur Einstellung der Lamellenstruktur 100 ist eine automatische Steuerung vorgesehen, die anhand eines vorgegebenen Programms die Lamellenstruktur 100 in den unterschiedlichen Zonen 102, 103, 104, 105, 106, 107 unabhängig von einander einstellt. Zusätzlich ist in jeder Zone im Bereich der Lamellenstruktur 100 ein Temperatursensor vorgesehen, der so mit der automatischen Steuerung verbunden ist, dass eine Einstellung der Lamellen 101 anhand der gemessenen Temperatur erfolgen kann.

Weiters weist die Personenliege 10 eine Abdeckung 17 auf. Diese kann mit einem Abstand zur Liegefläche 11 angeordnet werden, sodass zwischen Liegefläche 11 und Abdeckung 17 ein weiterer Dampfraum entsteht, der mit dem Dampfraum 12 verbunden ist.

Durch die dargestellte Luftbefeuchtungsvorrichtung 1 und die dargestellte Personen liege 10 kann eine besonders effektive Bedampfung erfolgen.

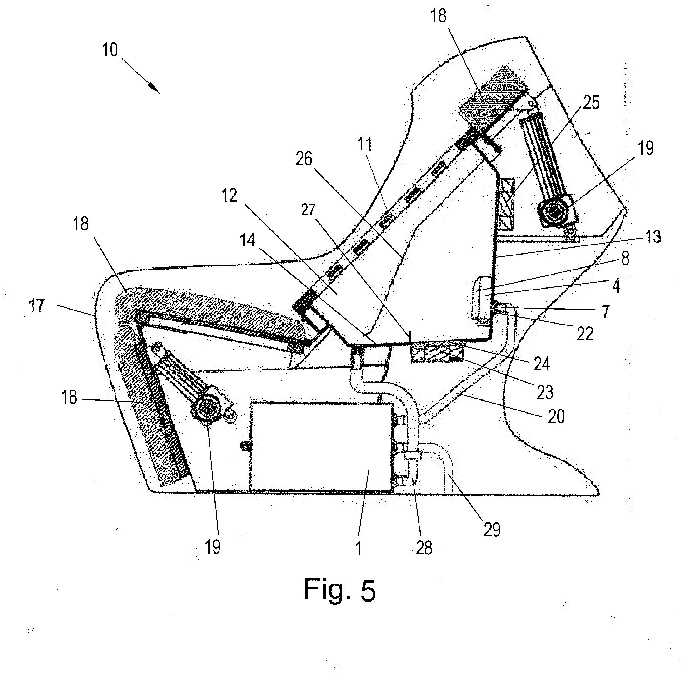

Fig. 5. zeigt eine alternative Personen liege 10. Die Liegefläche 11 ist im Rückenbereich als Liegerost ausgebildet. Im Kopfbereich, im Oberschenkenbereich und im Unterschenkelbereich sind jeweils Polsterauflagen 18 vorgesehen. Diese Personen liege 10 eignet sich somit für eine gezielte Bedampfung des Schulter und Rückenbereichs. Um eine gemütliche Liegeposition einstellen zu können, sind der Kopfbereich und der Unterschenkelbereich durch Antriebe 19 verstellbar. Unterhalb der Liegefläche 11 ist der Dampfraum 12 ausgebildet. Der Dampfraum 12 weist eine dampfundurchlässige Unterseite 14 auf. An der, in der Figur rechts dargestellten, dampfundurchlässigen Seitenwand 13 ist ein Wasserdampfventilator 22 vorgesehen, der direkt auf den Dampfaustritt 7 und vor dem Reservoir 4 angeordnet ist. Dies ermöglicht eine gezielte Steuerung von Lufttemperatur und Luftfeuchtigkeit im Dampfraum 12.

Um zu vermeiden, dass der Dampf und die heiße Luft zu Verbrennungen führen, ist eine Trennwand 26 vorgesehen, die den Dampfraum 12 von oben bis in einen Bereich unterhalb des Dampfaustritts 7 teilt. Entlang der dampfundurchlässigen Unterseite 14 ist ein Dampfdurchlass vorgesehen, der die Verteilung des Dampfes im gesamten Dampfraum 12 ermöglicht. Dampf der aus dem Reservoir 4 austritt steigt aufgrund der hohen Temperatur zunächst nach oben. Dort wird er durch den Ventilator 25 verwirbelt und gelangt durch den Dampfdurchlass in den Bereich der Liegefläche 11, wo er wieder nach oben aufsteigt und so den gesamten Rückenbereich bedampft. Entlang des Strömungsweges kommt es zu einem Abkühlen, sodass die Bedampfung jedenfalls ausschließlich mit Nassdampf erfolgt.

In der dampfundurchlässigen Unterseite 14 ist in der dargestellten Ausführungsform ein Frischluftventilator 23 vorgesehen, wobei über dem Frischluftventilator 23 eine Heizfläche 24 vorgesehen ist. Dadurch kann trockene, insbesondere angewärmte, Luft in den Dampfraum 12 eingeblasen werden. Um zu vermeiden, dass stark erhitzte Luft direkt zur Liegefläche 11 gelangen kann, kann eine weitere Trennwand 27 vorgesehen sein. Kondenswasser, das sich an der Trennwand 24 ablagert, kann durch die Wasserrückführung 28 wieder der Luftbefeuchtungsvorrichtung 1 zugeführt werden. Um die Befüllung des Verdampfers 1 zu vereinfachen ist in der dargestellten Ausführungsform ein Wasseranschluss 29 vorgesehen.

Im Betrieb wird das Wasser in der Luftbefeuchtungsvorrichtung 1 verdampft und gelangt durch die Dampfleitung 20 zum Dampfaustritt 7 und in das direkt darauffolgend angeordnete Reservoir 4. Im Reservoir 4 kann beispielsweise eine Rindendroge, insbesondere Rinde von Syzygium cumini, angeordnete werden, wobei die Rindendroge vorzugsweise vorher befeuchtet wurde. Dazu kann beispielsweise der Deckel 8 des Reservoirs 4 verschlossen werden. Auf diese Weise ist auch eine Extraktion unter Druck möglich.

Je nach Betriebsart kann die Menge des Wasserdampfs durch den Wasserdampfventilator 22 reguliert werden. In einem Saunabetrieb wird zunächst nur warme Luft durch den Frischluftventilator 23 in den Dampfraum 12 eingeblasen. Um einen Aufguss zu simulieren, kann durch den Wasserdampfventilator 22 zusätzlich schwallartig Dampf in den Dampfraum 12 abgegeben werden. Soll hingegen eine feuchte Bedampfung des Körpers stattfinden kann eine kontinuierliche Abgabe von Wasserdampf erfolgen.

### Beispiel

Bei einem Diabetes mellitus Typ 2 Patienten wurde an 6 aufeinanderfolgenden Tagen der morgendliche Nüchternblutzucker bestimmt. Anschließend wurde der Patient täglich abends mit fermentierter Rindendroge aus Syzygium cumini bedampft. Die Bedampfung erfolgte auf einer zuvor zu Fig. 2 beschriebenen Personenliege. Die Bedampfungsdauer wurde nach Bedarf des Patienten gewählt. Es wurde an den auf die abendliche Bedampfung folgenden Tagen ebenfalls der Nüchternblutzucker bestimmt.

An Tag 7 wurde die abendliche Bedampfung ausgesetzt. An Tag 9 wurde die üblicherweise eingenommene Medikation ausgesetzt. Die gemessenen Blutzuckerwerte sind in Tabelle 1 zusammengefasst.

**Tab.1**

| Behandlungstag | Blutzucker in mg/dl |
|---|---|
| 1 | 155 |
| 2 | 147 |
| 3 | 163 |
| 4 | 122 |
| 5 | 154 |
| 6 | 146 |
| 7 | 117 |
| 8 | 137 |
| 9 | 117 |
| 10 | 134 |
| 11 | 110 |
| 12 | 109 |
| 13 | 92 |
| 14 | 114 |

Neben den verbesserten Blutzuckerwerten wurde eine Verbesserung des Allgemeinbefindens festgestellt. So kam es zu vermindertem Kribbeln an den Füssen und zu einer verbesserten Thermoregulation.

## Patentansprüche

1. Personenliege (10), insbesondere Wellnessliege, umfassend eine dampfdurchlässige Liegefläche (11), wobei unterhalb der Liegefläche (11) ein Dampfraum (12) vorgesehen ist, und wobei die Personenliege (10) eine Luftbefeuchtungsvorrichtung (1) umfassend eine Wasserversorgungsvorrichtung (2), ein Heizelement zur Erzeugung von Wasserdampf, und einen Dampfauslass (3) zur Abgabe des erzeugten Dampfes in den Dampfraum (12) beinhaltet, wobei im Dampfauslass (3) oder in Dampfströmungsrichtung direkt auf den Dampfauslass (3) folgend ein Reservoir (5) zur Aufnahme einer Droge (4) vorgesehen ist, und wobei die Personenliege (10) **dadurch gekennzeichnet ist, dass** zwischen dem Dampfraum (12) und der Liegefläche (11) eine reversibel einstellbare Lamellenstruktur (100) zur Steuerung des Dampfdurchtritts vom Dampfraum (12) zur Liegefläche (11) angeordnet ist, wobei die Lamellenstruktur (100) kippbare Lamellen (101) aufweist.

2. Personenliege (10) nach Anspruch 1, wobei in dem Reservoir (5) eine Droge (4), insbesondere eine Rindendroge, vorzugsweise Rinde von Syzygium cumini, angeordnet ist.

3. Personenliege (10) nach Anspruch 1 oder 2, wobei die Lamellenstruktur (100) entlang der Liegefläche (11) mehrere Zonen (102, 103, 104, 105, 106, 107) aufweist, wobei die Zonen (102, 103, 104, 105, 106, 107) unabhängig voneinander einstellbar sind.

4. Personenliege (10) nach Anspruch 3, wobei die Lamellenstruktur (100) zumindest eine unabhängig einstellbare Kopfzone (102) am oberen Ende der Liegefläche (11) aufweist.

5. Personenliege (10) nach einem der Ansprüche 1 bis 4, wobei an der Lamellenstruktur (100), insbesondere in jeder Zone (102, 103, 104, 105, 106, 107) der Lamellenstruktur (100), zumindest ein Sensor zur Messung der Temperatur des vorbeiströmenden Wasserdampfs vorgesehen ist, und wobei die Personenliege (10) eine automatische Steuerung zur Einstellung der Lamellenstruktur (100) anhand der gemessenen Temperatur aufweist.

6. Personenliege nach einem der Ansprüche 1 bis 5, wobei die Personenliege (10) dampfundurchlässige Seitenteile (13) zur Begrenzung des unter der Liegefläche (11) angeordneten Dampfraums (12) aufweist.

7. Personenliege (10) nach einem der Ansprüche 1 bis 6, wobei die Personenliege (10) eine der Liegefläche (11) gegenüberliegende dampfundurchlässige Unterseite (14) zur Begrenzung des Dampfraums (12) aufweist, wobei insbesondere vorgesehen ist, dass die dampfundurchlässige Unterseite (14) beheizbar ist.

8. Personenliege (10) nach einem der Ansprüche 1 bis 7, wobei im Dampfraum (12), insbesondere in einer Fußzone des Dampfraums (12), ein regulierbarer Ventilator vorgesehen ist, wobei insbesondere vorgesehen ist, dass die Personenliege (100) eine automatische Steuerung zur Regulierung des Ventilators aufweist, wobei vorzugsweise vorgesehen ist, dass die Regulierung des Ventilators anhand der im Dampfraum (12) oder an der Lamellenstruktur (100) gemessenen Temperatur erfolgt.

9. Personenliege (10) nach einem der Ansprüche 1 bis 8, wobei oberhalb der Liegefläche (11) ein mit dem Dampfraum (12) in Verbindung stehender weiterer Dampfraum ausgebildet ist, wobei eine Abdeckung (17) zur zumindest teilweisen Abgrenzung des weiteren Dampfraums von der Umgebungsluft vorgesehen ist.

10. Personenliege (10) nach Anspruch 9, wobei die Abdeckung (17) zumindest eine einstellbare Abdeckungsöffnung zum Luftaustausch mit der Umgebung aufweist, wobei insbesondere vorgesehen ist, dass die Abdeckungsöffnung eine automatische Steuerung zur Einstellung der Abdeckungsöffnung aufweist, wobei vorzugsweise vorgesehen ist, dass die Einstellung der Abdeckungsöffnung anhand der an der Abdeckung (17) gemessenen Temperatur erfolgt.

## Claims

1. Bed for a person (10), in particular a wellness bed, comprising a vapour-permeable bed surface (11), wherein a steam chamber (12) is provided beneath the bed surface (11), and wherein the bed for a person (10) includes an air humidification device (1) comprising a water supply device (2), a heating element for generating steam and a steam outlet (3) for discharging the generated steam into the steam chamber (12),
wherein a reservoir (5) for receiving a drug (4) is provided in the steam outlet (3) or directly after the steam outlet (3) in the direction of flow of the steam, and wherein the bed for a person (10) is **characterised in that**
a reversibly adjustable lamella structure (100) for controlling the passage of steam from the steam chamber (12) to the bed surface (11) is arranged between the steam chamber (12) and the bed surface (11), wherein the lamella structure (100) has tiltable lamellas (101).

2. Bed for a person (10) according to claim 1, wherein a drug (4), in particular a bark drug, preferably Syzygium cumini bark, is arranged in the reservoir (5).

3. Bed for a person (10) according to claim 1 or 2, wherein the lamella structure (100) has a plurality of zones (102, 103, 104, 105, 106, 107) along the bed surface (11), wherein the zones (102, 103, 104, 105, 106, 107) can be adjusted independently of each other.

4. Bed for a person (10) according to claim 3, wherein the lamella structure (100) has at least one independently adjustable head zone (102) at the top end of the bed surface (11).

5. Bed for a person (10) according to any one of claims 1 to 4, wherein at least one sensor for measuring the temperature of the passing steam is provided on the lamella structure (100), in particular in each zone (102, 103, 104, 105, 106, 107) of the lamella structure (100), and wherein the bed for a person (10) has an automatic control system for adjusting the lamella structure (100) on the basis of the temperature measured.

6. Bed for a person according to any one of claims 1 to 5, wherein the bed for a person (10) has vapour-impermeable side sections (13) for delimiting the steam chamber (12) arranged beneath the bed surface (11).

7. Bed for a person (10) according to any one of claims 1 to 6, wherein the bed for a person (10) has a vapour-impermeable underside (14) opposite the bed surface (11) for delimiting the steam chamber (12), wherein provision is made, in particular, for the vapour-impermeable underside (14) to be heatable.

8. Bed for a person (10) according to any one of claims 1 to 7, wherein an adjustable fan is provided in the steam chamber (12), in particular in a foot zone of the steam chamber (12), wherein provision is made, in particular, for the bed for a person (100) to have an automatic control system for adjusting the fan, wherein provision is preferably made for adjustment of the fan to take place on the basis of the temperature measured in the steam chamber (12) or on the lamella structure (100).

9. Bed for a person (10) according to any one of claims 1 to 8, wherein a further steam chamber connected to the steam chamber (12) is formed above the bed surface (11), wherein a cover (17) is provided for at least partially delimiting the further steam chamber from the ambient air.

10. Bed for a person (10) according to claim 9, wherein the cover (17) has at least one adjustable cover opening for the exchange of air with the environment, wherein provision is made, in particular, for the cover opening to have an automatic control system for adjusting the cover opening, wherein provision is preferably made for adjustment of the cover opening to take place on the basis of the temperature measured on the cover (17).

## Revendications

1. Transat (10), en particulier transat de bien-être, comprenant une surface de couchage (11) perméable à la vapeur, dans lequel une chambre de vapeur (12) est prévue en dessous de la surface de couchage (11), et dans lequel le transat (10) contient un dispositif d'humidification de l'air (1) comprenant un dispositif d'alimentation en eau (2), un élément chauffant destiné à la génération de vapeur d'eau et une évacuation de vapeur (3) destinée à l'émission de la vapeur générée dans la chambre de vapeur (12),
dans lequel un réservoir (5) destiné à la réception d'une drogue (4) est prévu dans l'évacuation de vapeur (3) ou directement après l'évacuation de vapeur (3) dans la direction d'écoulement de vapeur, et dans lequel le transat (10) est **caractérisé en ce que**
entre la chambre de vapeur (12) et la surface de couchage (11) est disposée une structure lamellaire (100) réglable de manière réversible et destinée à la commande du passage de vapeur depuis la chambre de vapeur (12) vers la surface de couchage (11), dans lequel la structure lamellaire (100) présente des lamelles inclinables (101).

2. Transat (10) selon la revendication 1, dans lequel dans le réservoir (5) est disposée une drogue (4), en particulier une drogue d'écorce, de préférence de l'écorce de jamelonier.

3. Transat (10) selon la revendication 1 ou 2, dans lequel la structure lamellaire (100) présente le long de la surface de couchage (11) plusieurs zones (102, 103, 104, 105, 106, 107), dans lequel les zones (102, 103, 104, 105, 106, 107) sont réglables indépendamment les unes des autres.

4. Transat (10) selon la revendication 3, dans lequel la structure lamellaire (100) présente au moins un zone de tête (102) réglable indépendamment au niveau de l'extrémité supérieure de la surface de couchage (11).

5. Transat (10) selon l'une quelconque des revendications 1 à 4, dans lequel au moins un capteur destiné à la mesure de la température de la vapeur d'eau qui passe est prévu au niveau de la structure lamellaire (100), en particulier dans chaque zone (102, 103, 104, 105, 106, 107) de la structure lamellaire (100), et dans lequel le transat (10) présente une commande automatique destinée au réglage de la structure lamellaire (100) à l'aide de la température mesurée.

6. Transat selon l'une quelconque des revendications 1 à 5, dans lequel le transat (10) présente des parties latérales (13) imperméables à la vapeur et destinées à la délimitation de la chambre de vapeur (12) disposée sous la surface de couchage (11).

7. Transat (10) selon l'une quelconque des revendications 1 à 6, dans lequel le transat (10) présente une face inférieure (14) imperméable à la vapeur opposée à la surface de couchage (11) et destinée à la délimitation de la chambre de vapeur (12), dans lequel il est en particulier prévu que la face inférieure (14) imperméable à la vapeur puisse être chauffée.

8. Transat (10) selon l'une quelconque des revendications 1 à 7, dans lequel un ventilateur réglable est prévu dans la chambre de vapeur (12), en particulier dans une zone pour les pieds de la chambre de vapeur (12), dans lequel il est en particulier prévu que le transat (100) présente une commande automatique destinée au réglage du ventilateur, dans lequel il est de préférence prévu que le réglage du ventilateur s'effectue à l'aide de la température mesurée dans la chambre de vapeur (12) ou au niveau de la structure lamellaire (100).

9. Transat (10) selon l'une quelconque des revendications 1 à 8, dans lequel au-dessus de la surface de couchage (11) une autre chambre de vapeur en liaison avec la chambre de vapeur (12) est réalisé, dans lequel un couvercle (17) est prévu pour au moins la délimitation partielle de l'autre chambre de vapeur de l'air ambiant.

10. Transat (10) selon la revendication 9, dans lequel le couvercle (17) présente au moins une ouverture de couvercle réglable destinée à l'échange d'air avec l'environnement, dans lequel il est en particulier prévu que l'ouverture de couvercle présente une commande automatique destinée au réglage de l'ouverture de couvercle, dans lequel il est de préférence prévu que le réglage de l'ouverture de couvercle s'effectue à l'aide de la température mesurée au niveau du couvercle (17).
